# EUROPEAN PATENT SPECIFICATION

(11) **EP 0 746 272 B1**
(45) Date of publication and mention of the grant of the patent: **24.10.2001**
(21) Application number: 94927220.7
(22) Date of filing: 26.08.1994
(51) Int. Cl.: A61F 2/14

(54) **METHOD OF CHOOSING A DEVICE FOR ALTERING CORNEAL REFRACTIVE PROPERTIES**
VERFAHREN ZUR AUSWAHL EINER VORRICHTUNG ZUM MODIFIZIEREN DER BRECHUNGSEIGENSCHAFTEN DER HORNHAUT
METHODE POUR CHOISIR UN DISPOSITIF DESTINE A MODIFIER LES PROPRIETES DE LA REFRACTION DE LA CORNEE

(30) Priority: 26.08.1993 US 112177
(43) Date of publication of application: 11.12.1996
(73) Proprietor: KERAVISION, INC., Fremont, California 94538-7353 (US)
(72) Inventor: SILVESTRINI, Thomas, Alamo, CA 94507 (US); MATHIS, Mark, L., Fremont, CA 94539 (US); SCHOLL, John, A., Danville, CA 94506 (US); PROUDFOOT, Robert, A., Santa Clara, CA 95051 (US)
(74) Representative: Price, Nigel John King
(86) International application number: US9409560
(87) International publication number: WO9505789

(56) References cited:
- WO-A-93/13724
- WO-A-93/20763
- SU-A- 388 746
- US-A- 4 452 235
- US-A- 4 961 744
- US-A- 5 318 047

## Description

### Technical Field

This invention is in the general field of medical technology and relates specifically to a method of choosing a device for varying the curvature of the cornea of the eye in order to correct vision problems. The device is an intrastromal corneal ring which is inserted into the cornea and varies the radius of curvature and/or the aspheric shape of the cornea as a function of the cone angle of the device.

### Background

Anomalies in the shape of the eye can cause visual disorders. Axial hyperopia ("farsightedness") occurs when the front-to-back distance in the eyeball is too small. Curvature hyperopia occurs when the corneal curvature is less than normal and therefore is flatter than the normal cornea, In these cases, parallel rays originating greater than 6.1 metres (20 feet) from the eye focus behind the retina. In contrast, when the front-to-back distance of the eyeball is too large, axial myopia ("nearsightedness") occurs. When the corneal curvature is too great, curvature myopia occurs. In these cases, the focus of parallel rays entering the eye occurs in front of the retina. Astigmatism is a condition which occurs when the parallel rays of light do not focus to a single point within the eye, but rather have a variable focus due to the fact that the corneal curvature varies in different meridians. Light is therefore refracted different distances and focuses at different regions. Some degree of astigmatism is normal, but where astigmatism is too pronounced, it must often be corrected. Presbyopia is an age-related condition that results in the loss of the ability of the eye to change focal length.

Hyperopia, myopia, presbyopia and astigmatism are usually corrected by glasses or contact lenses. Surgical methods for the correction of such disorders have been cited in the literature and include radial keratotomy (see e.g. U.S. Patents Nos. 4,815,463 and 4,688,570) and laser corneal ablation (see e.g. U.S. Patent No. 4,941,093). Another method for correcting those disorders is through implantation of polymeric rings in the eye's corneal stroma to change the curvature of the cornea. Previous work involving the implantation of polymethylmethacrylate (PMMA) rings, allograft corneal tissue and hydrogels is well documented. One of the ring devices involves a ring design that allows a split ring to be inserted into a channel dissected in the stromal layer of the cornea. The device uses a minimally invasive incision through which the channel for the implant is created and through which the implant is inserted and adjusted. Adjustment of the device normally involves an adjustment of ring size or diameter.

U.S. Patent No. 4,452,235 describes a method and apparatus for corneal curvature adjustment. The method involves inserting one end of a split end adjusting ring into the cornea of the eye and moving the ring in a circular path until its ends meet. The ends are thereafter adjusted relative to each other until the shape of the eye has assumed a desired curvature whereupon the ends are fixedly attached to maintain the desired curvature of the cornea.

WO-A-93/13724 discloses a method for refractive correction of the eye in order to improve the vision of the eye while not adversely affecting its natural asphericity. The method involves determining the amount of correction necessary, selecting an intrastromal corneal ring of appropriate thickness to obtain the necessary correction (from a selection of intrastromal corneal rings of varying thickness) and inserting the selected intrastromal corneal ring into the corneal stroma.

### Summary of the Invention

According to the present invention there is provided a method of choosing an intrastromal corneal ring for insertion into the cornea of a patient's eye to, once inserted, substantially encircle the cornea and refractively to correct the eye for the purpose of improving the patient's vision, the intrastromal corneal ring having a cone angle of between about 0° and 50°, the method comprising selecting the ring based on its cone angle to effect corrective refraction of the patient's eye.

The hereinafter described and illustrated exemplary intrastromal corneal rings are for varying the curvature and/or the aspheric shape of the cornea of an eye, typically for the purpose of improving the vision of the eye. The exemplary intrastromal corneal rings are split polymeric rings and are adapted to substantially encircle the cornea. The intrastromal corneal rings preferably have a thickness of between about 0.05 and 0.60 mm and a cone angle above about 22.5°. The cone angle is chosen based on the starting curvature of the eye and on the thickness of the intrastromal corneal ring and the type of aspheric shape desired. The chosen exemplary intrastromal corneal ring may then be inserted into the corneal stroma to increase the radius of corneal curvature and/or change the aspheric shape of the cornea. Such a method may be useful in the treatment of myopia, presbyopia and astigmatism.

### Brief Description of the Drawings

Figure 1 is a schematic representation of a horizontal section of the eye.
Figure 2 is a schematic illustration of the anterior portion of the eye showing the various layers of the cornea.
Figure 3 is a schematic representation of an eye showing the average corneal curvature radius and aspheric shape of the cornea.
Figure 4 is a schematic representation of a myopic eye showing the average corneal curvature radius and the aspheric shape of the cornea.
Figure 5 is a plan view of an intrastromal corneal ring of the invention.
Figure 6 is an cross sectional view of an intrastromal corneal ring of the invention.
Figure 7 is a graph showing the cone angle "N" to be used with intrastromal corneal rings of varying thickness.
Figures 8A, 9A, 10A, 11A, 12A, 13A, and 14A are corneal maps that show the change in topography of the cornea when intrastromal corneal rings of differing thickness and a 34° cone angle are implanted into a cadaver eye. Figures 8B, 9B, 10B, 11B, 12B, 13B, and 14B are corneal maps that show the change in the topography of the cornea as observed in two different planes that are 90° apart.

Like elements in the drawings bear the same reference numerals.

### Modes for Carrying Out the Invention

Prior to explaining the details of the inventive method, a short explanation of the physiology of the eye is needed to appreciate the functional relationship of the device to the eye.

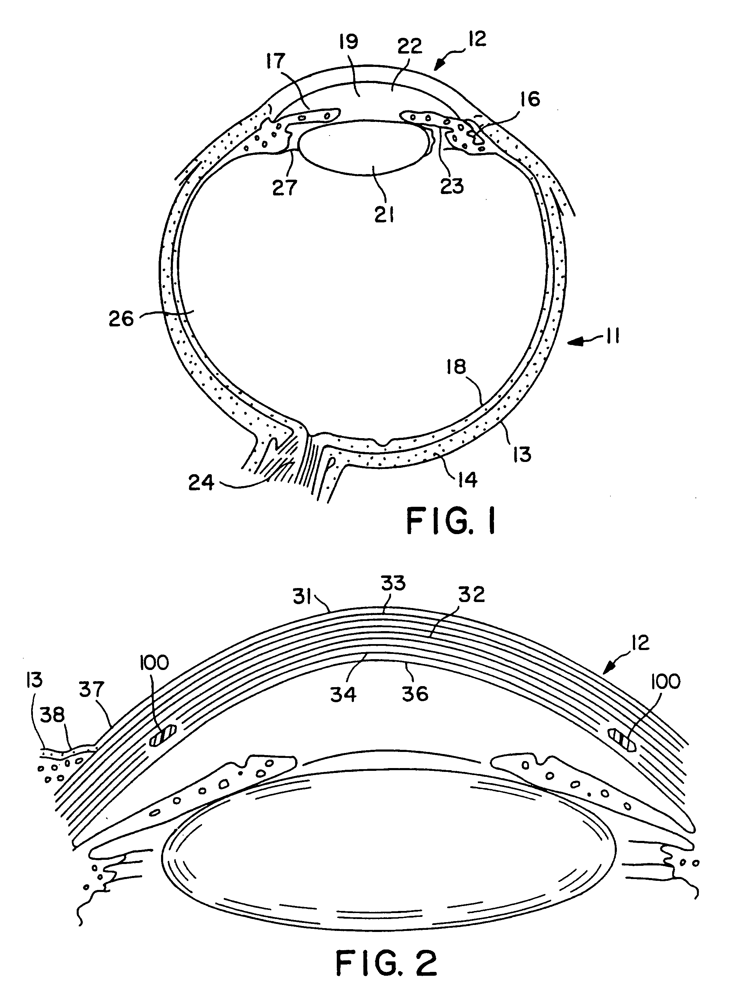

Figure 1 shows a horizontal section of the eye with the globe (11) of the eye resembling a sphere with an anterior bulged spherical portion representing the cornea (12).

The globe (11) of the eye consists of three concentric coverings enclosing the various transparent media through which the light must pass before reaching the sensitive retina (18). The outermost covering is a fibrous protective portion the posterior five-sixths of which is white and opaque and called the sclera (13), and sometimes referred to as the white of the eye where visible to the front. The anterior one-sixth of this outer layer is the transparent cornea (12).

A middle covering is mainly vascular and nutritive in function and is comprised of the choroid (14), ciliary body (16) and iris (17). The choroid (14) generally functions to maintain the retina (18). The ciliary body (16) is involved in suspending the lens (21) and accommodation of the lens. The iris (17) is the most anterior portion of the middle covering of the eye and is arranged in a frontal plane. It is a thin circular disc corresponding to the diaphragm of a camera, and is perforated near its center by a circular aperture called the pupil (19). The size of the pupil varies to regulate the amount of light which reaches the retina (18). It contracts also to accommodation, which serves to sharpen the focus by diminishing spherical aberration. The iris (17) divides the space between the cornea (12) and the lens (21) into an anterior chamber (22) and posterior chamber (23). The innermost portion of covering is the retina (18), consisting of nerve elements which form the true receptive portion for visual impressions.

The retina (18) is a part of the brain arising as an outgrowth from the fore-brain, with the optic nerve (24) serving as a fiber tract connecting the retina part of the brain with the fore-brain. A layer of rods and cones, lying just beneath a pigmented epithelium on the anterior wall of the retina serve as visual cells or photoreceptors which transform physical energy (light) into nerve impulses.

The vitreous body (26) is a transparent gelatinous mass which fills the posterior four-fifths of the globe (11). At its sides it supports the ciliary body (16) and the retina (18). A frontal saucer-shaped depression houses the lens.

The lens (21) of the eye is a transparent biconvex body of crystalline appearance placed between the iris (17) and vitreous body (26). Its axial diameter varies markedly with accommodation. A ciliary zonule (27), consisting of transparent fibers passing between the ciliary body (16) and lens (21) serves to hold the lens (21) in position and enables the ciliary muscle to act on it.

Referring again to the cornea (12), this outermost fibrous transparent coating resembles a watch glass. Its curvature is somewhat greater than the rest of the globe and is ideally spherical in nature. However, often it is more curved in one meridian than another giving rise to astigmatism. A central third of the cornea is called the optical zone with a slight flattening taking place outwardly thereof as the cornea thickens towards its periphery. Most of the refraction of the eye takes place through the cornea.

Referring to Figure 2, a more detailed drawing of the anterior portion of the globe shows the various layers of the cornea (12) comprising an epithelium (31). Epithelial cells on the surface thereof function to maintain transparency of the cornea (12). These epithelial cells are rich in glycogen, enzymes and acetylcholine and their activity regulates the corneal corpuscles and controls the transport of water and electrolytes through the lamellae of the stroma (32) of the cornea (12).

An anterior limiting lamina (33), referred to as Bowman's membrane or layer, is positioned between the epithelium (31) and the stroma (32) of the cornea. The stroma (32) is comprised of lamella having bands of fibrils parallel to each other and crossing the whole of the cornea. While most of the fibrous bands are parallel to the surface, some are oblique, especially anteriorly. A posterior limiting lamina (34) is referred to as Descemet's membrane. It is a strong membrane sharply defined from the stroma (32) and resistant to pathological processes of the cornea.

The endothelium (36) is the most posterior layer of the cornea and consists of a single layer of cells. The limbus (37) is the transition zone between the conjunctiva (38) and sclera (13) on the one hand and the cornea (12) on the other.

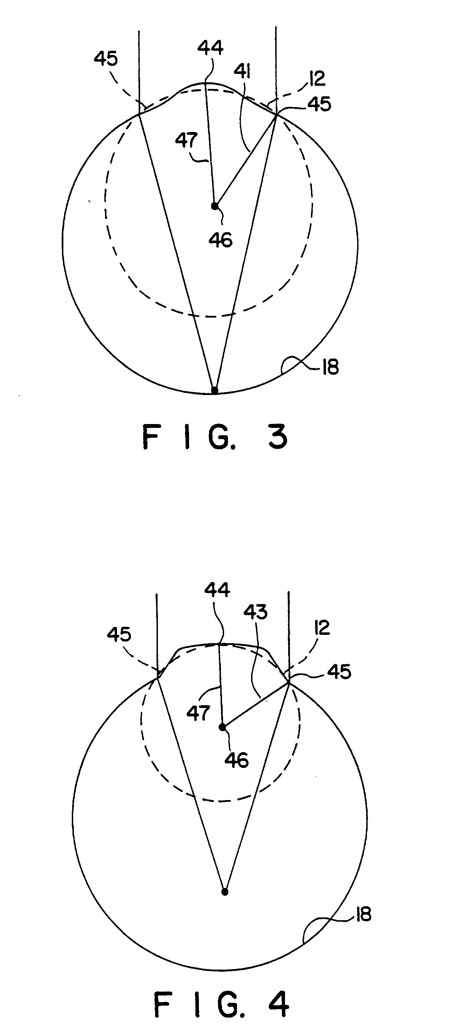

Figure 3 shows the globe of the eye having a cornea (12) with an average spherical radius of curvature (41) and a positive aspheric shape. By "average spherical radius of curvature" we intend the radius of the circle defined by the points at the periphery (45) of the cornea near the limbus of the eye and having a center (46). By "positive aspheric shape" we mean that the distance (47) from that center (46) to the anterior center of the cornea is greater than the average spherical radius of curvature, that is, the anterior surface of the cornea flattens as it progresses from the center (44) to its periphery (45). As shown in Figure 3, when parallel rays of light pass through the corneal surface, they are refracted by the corneal surfaces to converge eventually near the retina (18) of the eye. The diagram of Figure 3 discounts, for the purposes of this discussion, the refractive effect of the lens or other portions of the eye.

The eye depicted in Figure 4 is myopic because the light rays from the periphery of the cornea refract into focus at a point in the vitreous body which is in front of the retinal surface. Further, the eye depicted in Figure 4 has does not have the same aspheric shape as that shown in Figure 3. The distance (47) from the center (46) to the anterior surface of the cornea is about the same as or less than the average spherical radius of curvature (41) and thus the cornea does not flatten from center (44) to periphery (45) but rather plateaus or even dips at its center. If an intrastromal is implanted into the cornea shown in Figure 4, the light rays refracted by the now flattened corneal surface will be refracted at a smaller angle and thus converge at a more distant point such as directly on the retina. Further, selection of an intrastromal corneal ring having an appropriate cone angle may allow for the eye to obtain a more positive aspheric shape similar to that shown in the Figure 3 eye.

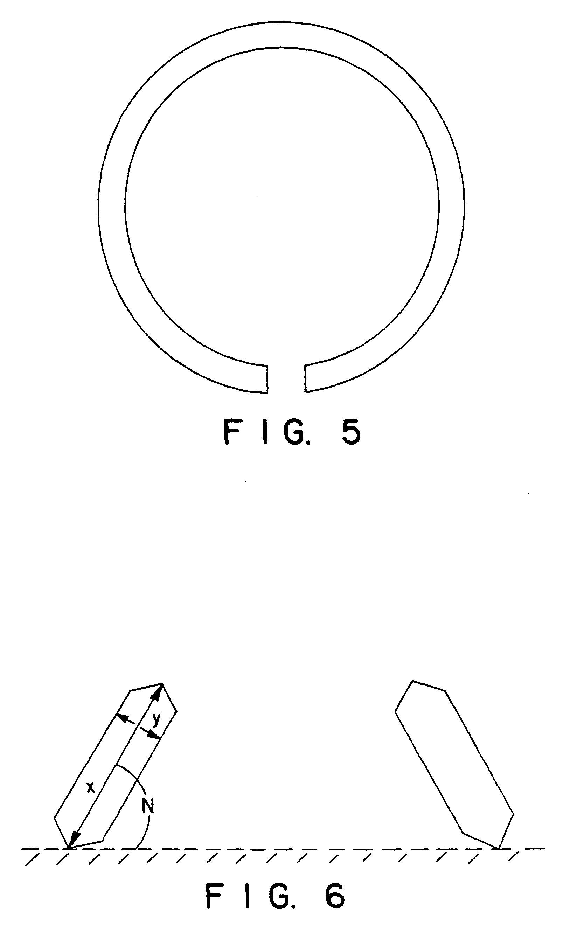

With the background discussion of Figures 1-4 in hand, it should be understood that the device chosen according to the method of the present invention is for the adjustment of at least a portion of an annular chord of the cornea to decrease the radius of curvature and/or change the shape of the cornea in order to improve the vision of the eye. We have found that the shape of the anterior corneal surface may be adjusted by using intrastromal corneal rings having varying "cone angles". The cone angle "N" of the intrastromal corneal ring is shown as angle N in Figure 6. Cone angle "N" is defined as the angle between the plane of the flat surface that the intrastromal corneal ring rests on and a line drawn between the point of the cross-section that rests on the flat surface and the point on the cross-section that is farthest from the point where the intrastromal corneal ring rests on the flat surface. The cross-section referred to is one that cuts through the diameter of the intrastromal corneal ring and is at an angle of 90° to the flat surface.

Previously, the cone angle N was generally considered appropriate at a fixed value equal to about 22.5°. This angle was selected to be one which would generally be tangential to the corneal anterior surface at the front surface of the installed intrastromal corneal ring. We have now found that by varying the cone angle, the rate of change of the radius of curvature of the cornea progressing from the center to the periphery of the cornea can be adjusted such that the'cornea assumes an aspheric profile that may closely match the positive aspheric shape shown in Figure 3, depending on the cone angle chosen and the natural shape of the cornea. The cone angle N will be above 22.5°, preferably it will be between about 23° and 50°, preferably it will be between about 23° and 35°. Such a ring placed, for instance, approximately at the 8 mm chord of the cornea provides a means for adjusting the radius of curvature of the cornea so that vision is improved. Other placements will depend on the physical dimensions of the particular eye to be implanted.

By selecting the thickness of the ring according to the amount of corrective refraction desired, the rays refracted by the cornea and other eye components may be brought to focus directly on the retina. The cone angle N may be selected to maintain the aspheric shape of the eye prior to insertion of the ring. In other cases, the cone angle N may be selected to alter the aspheric shape of the eye.

Figure 5 shows one desirable intrastromal corneal ring for to the invention. The intrastromal corneal ring is comprised of a generally circular member having split end portions. The material should have properties that render it physiologically compatible with the tissue of the cornea. An illustrative material is a plastic type material sold under the trade name of PERSPEX CQ™ (Imperial Chemical Company, England), however many other biocompatible polymers including but not limited to Teflon (Registered Trade Mark), PMMA and polysulfones are useful in the invention. One acceptable cross sectional shape of the rings is the hexagonal shape shown in Figure 6 and is generally dimensioned to be about 0.5 mm to 2.0 mm from point to point (dimension "x") and from about 0.05 mm to 0.60 mm in thickness (dimension "y"). Rings of other cross-sectional shapes including but not limited to ovoloid and rectangular shapes may be useful in the invention as well.

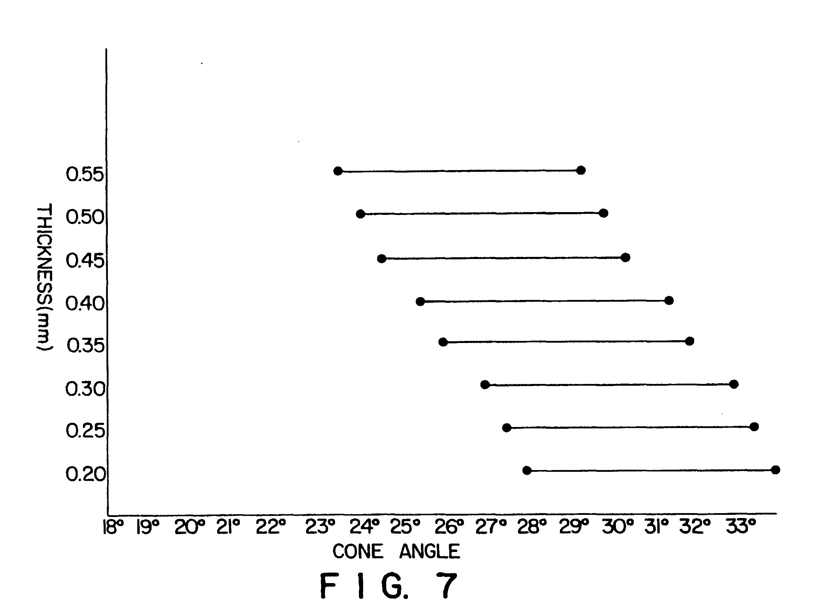

Even where the eye is not myopic, the method of the present invention may be useful to choose a device for changing the radius of curvature of the cencral cornea and the aspheric shape of the cornea. Generally speaking, where it is desirable to obtain a flatter central corneal curvature without changing intrastromal corneal ring thickness, an intrastromal corneal ring with a higher degree cone angle (e.g. 34° rather than 25°) will be implanted. Where, however it is desirable to maintain the aspheric shape existing prior to insertion of the ICR, an ICR with an appropriate cone angle should be employed, particularly as the intrastromal corneal ring thickness is increased and the curvature of the eye is flattened overall. One example of a guide to intrastromal corneal rings having coordinated cone angles and related intrastromal corneal ring thicknesses is shown in Figure 7. Insertion of such rings into a "theoretical 43 diopter" eye provides correction between -0.5 and -13.5 diopters without significantly altering the asphericity of the eye. However, the relationship of cone angle to thickness may vary from eye to eye since the initial diameter and corneal curvature of the eye may also vary. According to Figure 7, where the thickness of the intrastromal corneal ring for example is 0.20 mm, the cone angle will be between about 28° and 34°, for 0.25 mm, the cone angle will be between about 27.5° and 33.5°, for 0.30 mm, the cone angle will be between about 27° and 33°, for 0.35 mm, the cone angle will be between about 26° and 32°, for 0.40 mm, the cone angle will be between about 25.5° and 31.5°, for 0.45 mm, the cone angle will be between about 24.5° and 30.5°, for 0.50 mm, the cone angle will be between about 24° and 30°, and where the thickness of the intrastromal corneal ring is 0.55 mm, the cone angle will be between about 23.5° and 29.5°.

In the method of the invention of choosing a device for the correction of myopia, the physician will determine the amount of corrective refraction necessary to improve a patient's vision. From the determination of the necessary corrective refraction, the physician will choose an intrastromal corneal ring with the appropriate thickness and cone angle to obtain the corneal curvature radius desired. The thickness will be between about 0.05 mm and 0.60 mm and the cone angle will be between 0° and 50°, preferably between about 18° and 50°, or between about 23° and 50°, or between about 23° and 35°. The ring may be installed in the inner lamellar regions of the corneal stroma by any of the methods we have shown in the past to be suitable for such installation. Particularly desired is the process and its allied apparatus shown in WO-A-93/20763. This document (WO-A-93/20763) is prior art against the present application under Art 54(3) EPC. In general, the ring is installed in the following manner: A small radial incision is made at the radius in which the ring is ultimately to be installed about the cornea. A dissector in the form of a split ring and having a point suitable for producing an interlamellar channel or tunnel in the corneal stroma is introduced through the small incision and rotated in such a fashion that a generally circular channel is formed completely about the cornea. The dissector is then rotated in the opposite direction to withdraw it from the tunnel thus formed. The intrastromal corneal ring is then introduced into the circular channel. Similar methodology will be used for the treatment of astigmatism and presbyopia.

In a further method for the correction of myopia, the physician will determine the amount of corrective refraction necessary to improve a patient's vision and from this determination will choose an ICR with the appropriate thickness to obtain the corneal curvature radius desired. In addition, the cone angle of the intrastromal corneal ring is chosen to change the aspheric shape such that the cornea flattens more from center to periphery. The cone angle and thickness is as described above. The ring may be installed in the inner lamellar regions of the corneal stroma as described above.

In yet another method for the correction of myopia, the physician will determine the amount of corrective refraction necessary to improve a patient's vision and from this determination will choose an intrastromal corneal ring with the appropriate thickness to obtain the corneal curvature radius desired. In addition, the cone angle is chosen to maintain the aspheric shape of the eye close to prior to implantation of the intrastromal corneal ring. Again, the cone angle and thickness of the ring are as described above and the ring may be installed in the inner lamellar regions of the corneal stroma as described above.

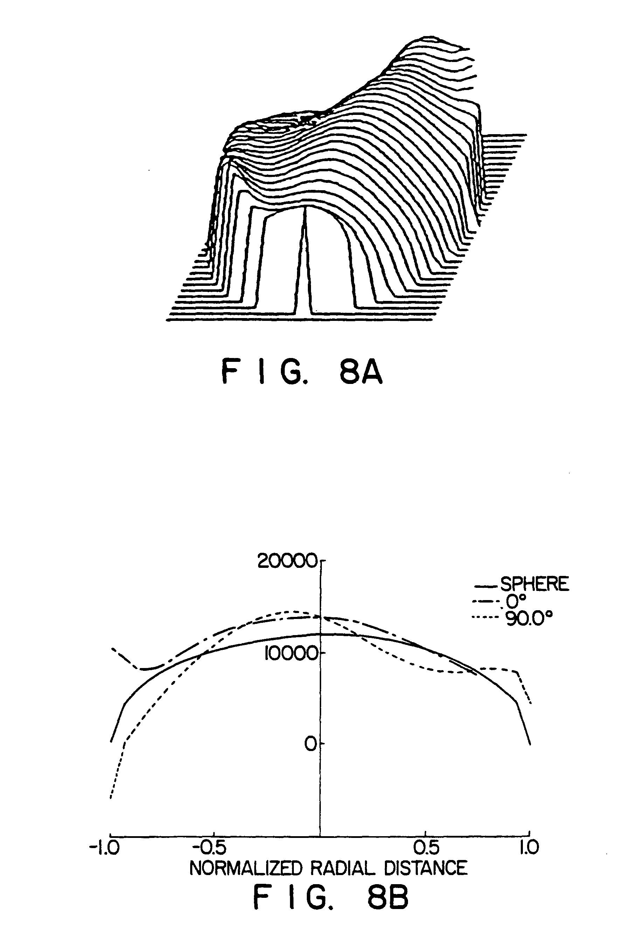

In yet one more method for the corrective refraction of an eye, where it is desirable to obtain or maintain a particular aspheric shape, the intrastromal corneal ring of particular thickness and cone angle may be implanted and the aspheric shape measured by using, for instance, a Kerametrics Class II Corneal Surface Analyzer. The instrument measures the corneal surface in terms of deviation from a spherical shape of a particular size. In Figures 8A, 9A, 10A, 11A, 12A, 13A, and 14A, measurement of a sphere would result in a graph having a rounded plateau or "mesa". Any deviation from the spherical baseline shows up on that three-dimensional depiction as a variance from that plateau. Figures 8B, 9B, 10B, 11B, 12B, 13B and 14B show both the shape of the baseline "sphere" and the deviation from the spherical baseline in two-dimensional depictions. Where the measured aspheric shape is not as desired, the intrastromal corneal ring will be removed and the intrastromal corneal ring with appropriate cone angle will be implanted.

The following Examples are intended to further illustrate but not to limit the invention in any manner. In each of the Examples below, the cross-sectional shape of the intrastromal corneal ring was hexagonal and the intrastromal corneal ring ends were not joined.

### Example 1

This Example shows the relationship between intrastromal corneal ring thickness and cone angle and the variation of the shape of the anterior corneal surface when an eye is implanted with intrastromal corneal ring of varying thickness that have the same cone angles.

In order to define the corneal topography that results from the intrastromal corneal ring cone angle effect, intrastromal corneal rings with thicknesses of 0.25, 0.30, 0.35 and 0.40 mm with constant cone angles of 34° were implanted into a deturgesced cadaver eye. The corneal topography of the eye was measured before and after intrastromal corneal ring implantation using the Kerametrics Class II Corneal Surface Analyzer. The Analyzer uses laser holographic interferometry to measure corneal topography.

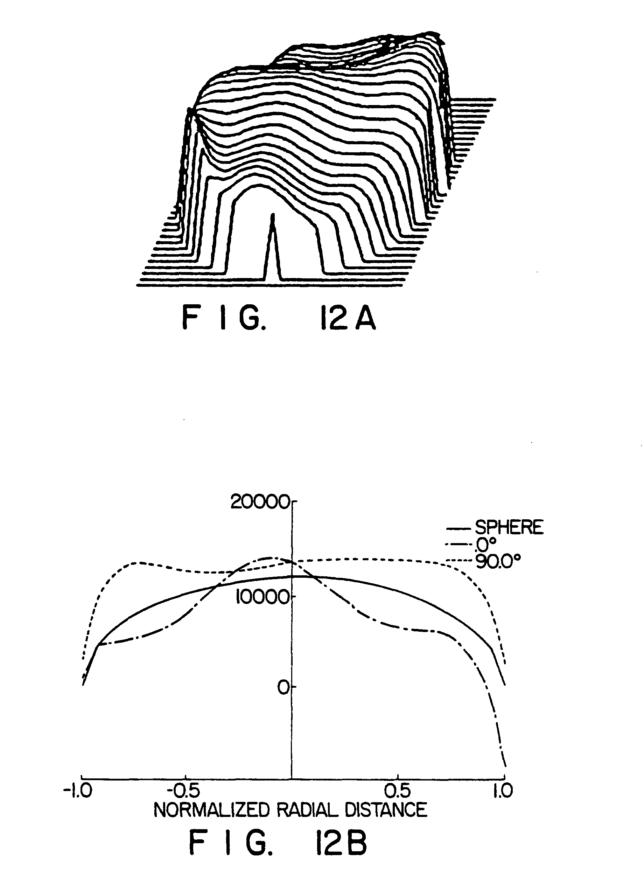

Figures 8-12 A and B show the corneal topography of an eye prior to and after being implanted with intrastromal corneal rings of varying thickness. Figure 8A shows the aspherical topography in three dimensions and Figure 8B shows the topography as observed in two different planes that are 90° apart. Both Figures show the topography of the eye in terms of deviation from a spherical shape before implantation of an intrastromal corneal ring. As is evident from the top right portion of Figure 8A, the eye has a nonsymmetrical aspheric shape, but it is normal in that the corneal curvature generally flattens from the center to the periphery.

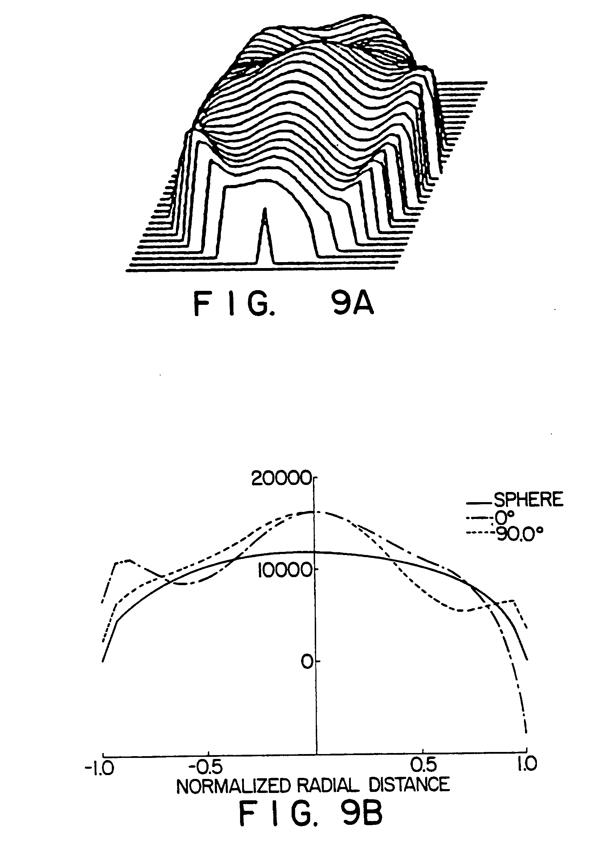

Figures 9A and 9B show the eye after implantation with a 0.25 mm thick intrastromal corneal ring. The aspheric shape has been maintained close to the original aspheric shape in what is considered to be a normal aspheric shape in that the curvature flattens from the center to the periphery.

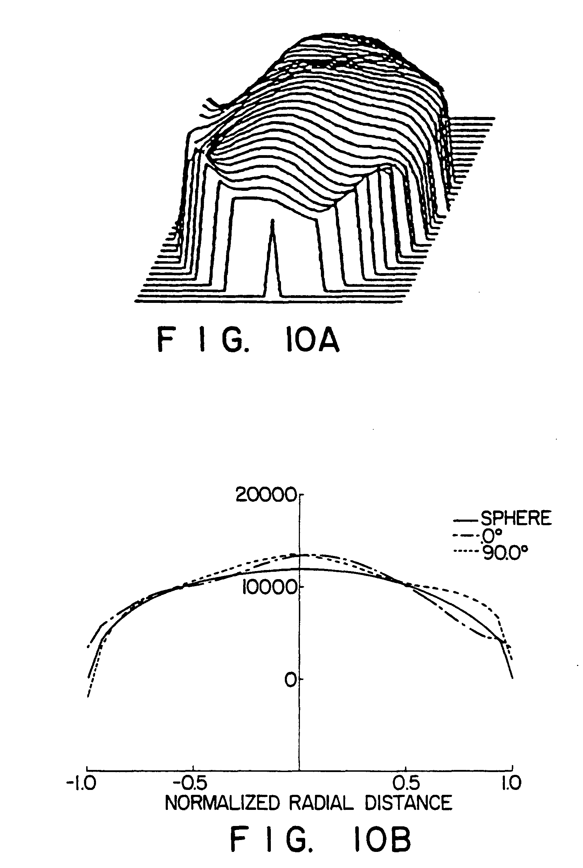

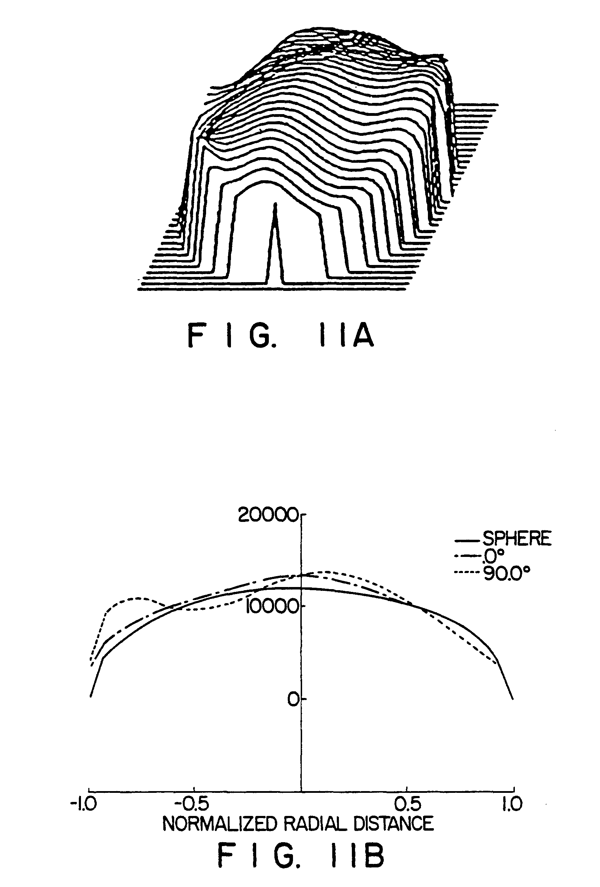

Figures 10A and 10B show the eye after implantation with a 0.30 mm thick intrastromal corneal ring. Figures 11A and 11B show the eye after implantation with a 0.35 mm thick intrastromal corneal ring. Implantation of both the 0.30 mm and 0.35 mm thick intrastromal corneal rings produce less curved but still acceptable aspheric shapes. Figures 12A and 12B show the eye after implantation with a 0.40 mm thick intrastromal corneal ring. These figures show an aspheric shape that does not flatten from center to periphery but bulges in the upper quadrant shown in Figure 12A. Such result indicates that an intrastromal corneal ring with a cone angle below 34° should be employed to produce a aspheric shape that flattens from center to periphery.

In sum, Figures 8-12 A and B indicate that the degree of change of the central corneal curvature can be carefully tuned depending on the cone angle and thickness of the intrastromal corneal ring chosen.

### Example 2

This Example shows the relationship between intrastromal corneal ring thickness and cone angle and the variation of the anterior corneal surface based on constant thickness and varying cone angle.

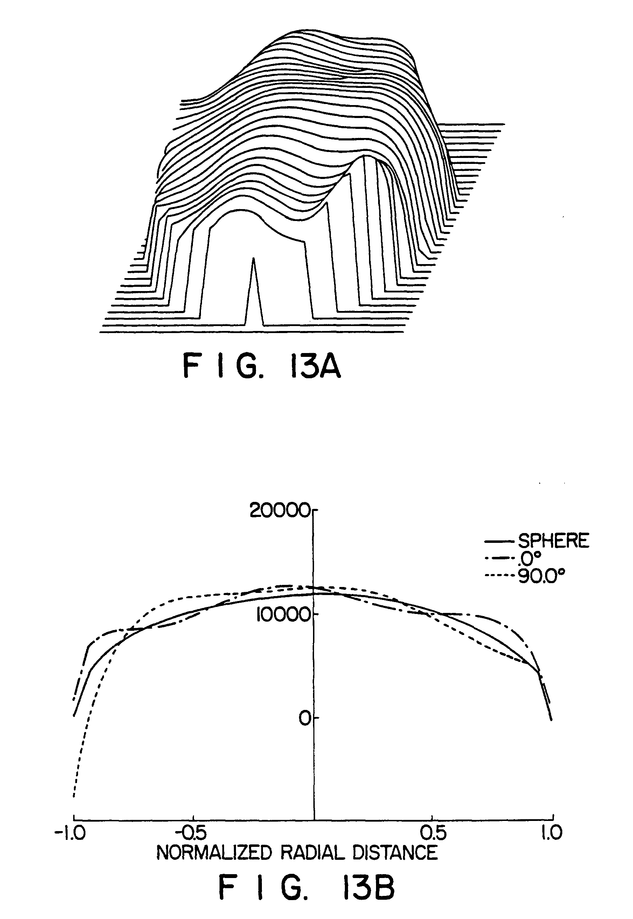

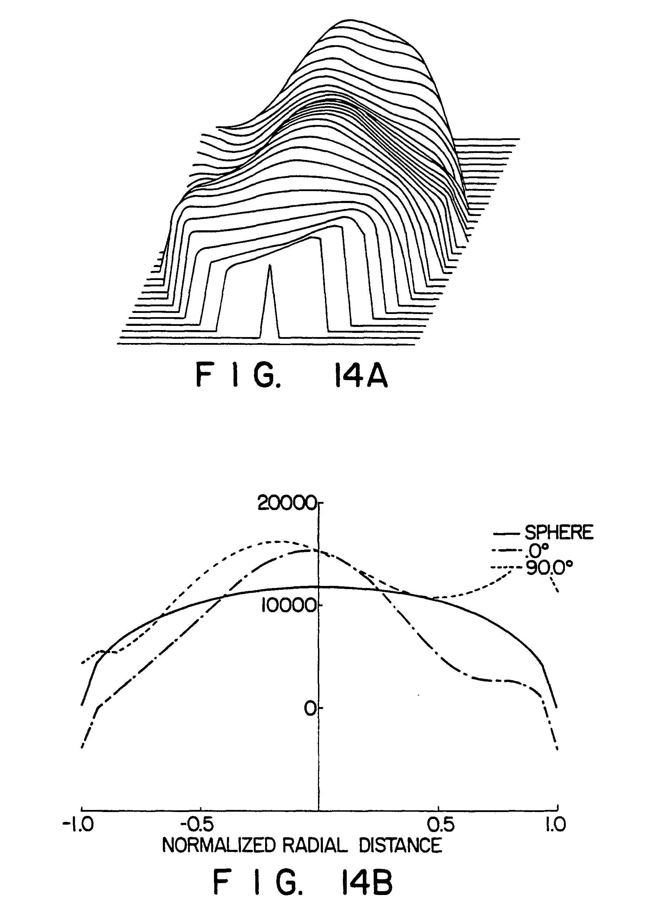

A 0.40 mm thick intrastromal corneal ring with a 25° cone angle was inserted into the eye. The corneal topography of the eye is shown in Figures 13A and 13B. Introduction of the intrastromal corneal ring produced a desirable aspheric shape. Removal of the 25°, 0.40 mm intrastromal corneal ring and replacement with a 34°, 0.40 mm ICR resulted in a much flatter central cornea (see Figures 14A and 14B). Therefore, to produce an aspheric shape that flattens more from center to periphery, an intrastromal corneal ring with a smaller cone angle would be used for this eye.

Modifications of the above described modes for carrying out the invention that are obvious to persons of skill in the fields of medicine, ophthalmology, optometry and/or related fields are intended to be within the scope of the following claims.

## Claims

1. A method of choosing an intrastromal corneal ring for insertion into the cornea of a patient's eye to, once inserted, substantially encircle the cornea and refractively to correct the eye for the purpose of improving the patient's vision, the intrastromal corneal ring having a cone angle (N) of between about 0° and 50°, the method comprising selecting the ring based on its cone angle to effect corrective refraction of the patient's eye.

2. The method of claim 1, comprising a previous step of determining the amount of corrective refraction necessary to improve the patient's vision.

3. The method of claim 1 or claim 2, wherein the intrastromal corneal ring is a split polymeric ring and has a cone angle (N) above about 22.5°.

4. The method of claim 1 or claim 2, wherein the cone angle (N) is selected to change the radius of curvature of the eye.

5. The method of claim 1 or claim 2, wherein the cone angle (N) is selected to change the aspheric shape of the cornea of the eye.

6. The method of claim 1 or claim 2, wherein the cone angle (N) is selected to maintain the asphericity of the eye close to the asphericity of the eye prior to insertion of the ring.

7. The method of any one of claims 4 to 6, wherein the intrastromal corneal ring has a thickness (y) of between about 0.05 and 0.60 mm.

8. The method of either of claims 1 and 2, wherein the cone angle (N) is between about 23° and 50°.

9. The method of either of claims 1 and 2, wherein the cone angle (N) is between about 18° and 50°.

10. The method of either of claims 1 and 2, wherein the thickness (y) of the ring is 0.20 mm and the cone angle (N) is between about 28° and 34°.

11. The method of either of claims 1 and 2, wherein the thickness (y) of the ring is 0.25 mm and the cone angle (N) is between about 27.5° and 33.5°.

12. The method of either of claims 1 and 2, wherein the thickness (y) of the ring is 0.30 mm and the cone angle (N) is between about 27° and 33°.

13. The method of either of claims 1 and 2, wherein the thickness (y) of the ring is 0.35 mm and the cone angle (N) is between about 26° and 32°.

14. The method of either of claims 1 and 2, wherein the thickness (y) of the ring is 0.40 mm and the cone angle (N) is between about 25.5° and 31.5°.

15. The method of either of claims 1 and 2, wherein the thickness (y) of the ring is 0.45 mm and the cone angle (N) is between about 24.5° and 30.5°.

16. The method of either of claims 1 and 2, wherein the thickness (y) of the ring is 0.50 mm and the cone angle (N) is between about 24° and 30°.

17. The method of either of claims 1 and 2, wherein the thickness (y) of the ring is 0.55 mm and the cone angle (N) is between about 23.5° and 29.5°.

## Patentansprüche

1. Verfahren zum Auswählen eines intraparenchymatösen Hornhautrings zum Einsetzen in die Hornhaut des Auges eines Patienten, damit er im eingesetzten Zustand die Hornhaut im wesentlichen umgibt und die Brechkraft des Auges korrigiert, um dadurch das Sehvermögen des Patienten zu verbessern, wobei der intraparenchymatöse Hornhautring einen Kegelwinkel (N) zwischen etwa 0° und 50° aufweist, umfassend das Auswählen des Rings gemäß seinem Kegelwinkel, um eine Korrekturrefraktion des Auges des Patienten zu bewirken.

2. Verfahren nach Anspruch 1, umfassend einen Vorab-Schritt des Bestimmens des Maßes der zur Verbesserung des Sehvermögens des Patienten benötigten Korrekturrefraktion.

3. Verfahren nach Anspruch 1 oder 2, bei dem der intraparenchymatöse Hornhautring ein Polymer-Spaltring ist und ein Kegelwinkel (N) von mehr als etwa 22,5° aufweist.

4. Verfahren nach Anspruch 1 oder 2, bei dem der Kegelwinkel (N) so gewählt wird, daß der Krümmungsradius des Auges geändert wird.

5. Verfahren nach Anspruch 1 oder 2, bei dem der Kegelwinkel (N) so gewählt wird, daß die aspherische Form der Hornhaut des Auges geändert wird.

6. Verfahren nach Anspruch 1 oder 2, bei dem der Kegelwinkel (N) so gewählt wird, daß der aspherische Zustand des Auges nahe bei dem aspherischen Zustand des Auges vor dem Einsetzen des Rings bleibt.

7. Verfahren nach einem der Ansprüche 4 bis 6, bei dem der intraparenchymatöse Hornhautring eine Dicke (y) von zwischen etwa 0,05 und 0,60 mm besitzt.

8. Verfahren nach Anspruch 1 oder 2, bei dem der Kegelwinkel (N) zwischen etwa 23° und 50° liegt.

9. Verfahren nach Anspruch 1 oder 2, bei dem der Kegelwinkel (N) zwischen etwa 18° und 50° liegt.

10. Verfahren nach Anspruch 1 oder 2, bei dem die Dicke (y) des Rings 0,20 mm und der Kegelwinkel (N) zwischen etwa 28° und 34° beträgt.

11. Verfahren nach Anspruch 1 oder 2, bei dem die Dicke (y) des Rings 0,25 mm beträgt und der Kegelwinkel (N) zwischen etwa 27,5°und 33,5° liegt.

12. Verfahren nach Anspruch 1 oder 2, bei dem die Dicke (y) des Rings 0,30 mm beträgt und der Kegelwinkel (N) zwischen etwa 27° und 33° liegt.

13. Verfahren nach Anspruch 1 oder 2, bei dem die Dicke (y) des Rings 0,35 mm beträgt und der Kegelwinkel (N) zwischen etwa 26° und 32° liegt.

14. Verfahren nach Anspruch 1 oder 2, bei dem die Dicke (y) des Rings 0,40 mm beträgt und der Kegelwinkel (N) zwischen etwa 25,5° und 31,5° liegt.

15. Verfahren nach Anspruch 1 oder 2, bei dem die Dicke (y) des Rings 0,45 mm beträgt und der Kegelwinkel (N) zwischen etwa 24,5° und 30,5° liegt.

16. Verfahren nach Anspruch 1 oder 2, bei dem die Dicke (y) des Rings 0,50 mm beträgt und der Kegelwinkel (N) zwischen etwa 24° und 30° liegt.

17. Verfahren nach Anspruch 1 oder 2, bei dem die Dicke (y) des Rings 0,55 mm beträgt und der Kegelwinkel (N) zwischen 23,5° und 29,5° liegt.

## Revendications

1. Méthode permettant de choisir un anneau cornéen intrastromal destiné à être inséré dans la cornée de l'oeil d'un patient afin qu'après insertion, l'anneau encercle substantiellement la cornée et corrige la qualité réfractive de l'oeil pour améliorer la vision du patient, l'anneau cornéen intrastromal ayant un angle conique (N) compris entre environ 0° et 50°, la méthode comprenant le fait de choisir l'anneau en fonction de son angle conique pour corriger la réfraction de l'oeil du patient.

2. Méthode selon la revendication 1, comprenant une étape préalable qui consiste à déterminer le degré de correction de la réfraction nécessaire pour améliorer la vision du patient.

3. Méthode selon la revendication 1 ou 2, dans laquelle l'anneau cornéen intrastromal est un anneau en polymère fendu, et présente un angle conique (N) supérieur à environ 22,5°.

4. Méthode selon la revendication 1 ou 2, dans laquelle l'angle conique (N) est choisi de façon à modifier le rayon de courbure de l'oeil.

5. Méthode selon la revendication 1 ou 2, dans laquelle l'angle conique (N) est choisi de façon à modifier la forme asphérique de la cornée de l'oeil.

6. Méthode selon la revendication 1 ou 2, dans laquelle l'angle conique (N) est choisi de façon à ce que la forme asphérique de l'oeil soit la plus proche de celle que présentait l'oeil avant l'insertion de l'anneau.

7. Méthode selon l'une quelconque des revendications 4 à 6, dans laquelle l'anneau cornéen intrastromal présente une épaisseur (y) comprise entre environ 0,05 et 0,60 mm.

8. Méthode selon l'une des revendications 1 ou 2, dans laquelle l'angle conique (N) est compris entre environ 23° et 50°.

9. Méthode selon l'une des revendications 1 ou 2, dans laquelle l'angle conique (N) est compris entre environ 18° et 50°.

10. Méthode selon l'une des revendications 1 ou 2, dans laquelle l'épaisseur (y) de l'anneau est de 0,20 mm et l'angle conique (N) est compris entre environ 28° et 34°.

11. Méthode selon l'une des revendications 1 ou 2, dans laquelle l'épaisseur (y) de l'anneau est de 0,25 mm et l'angle conique (N) est compris entre environ 27,5° et 33,5°.

12. Méthode selon l'une des revendications 1 ou 2, dans laquelle l'épaisseur (y) de l'anneau est de 0,30 mm et l'angle conique (N) est compris entre environ 27 ° et 33°.

13. Méthode selon l'une des revendications 1 ou 2, dans laquelle l'épaisseur (y) de l'anneau est de 0,35 mm et l'angle conique (N) est compris entre environ 26° et 32°.

14. Méthode selon l'une des revendications 1 ou 2, dans laquelle l'épaisseur (y) de l'anneau est de 0,40 mm et l'angle conique (N) est compris entre environ 25,5° et 31,5°.

15. Méthode selon l'une des revendications 1 ou 2, dans laquelle l'épaisseur (y) de l'anneau est de 0,45 mm et l'angle conique (N) est compris entre environ 24,5° et 30,5°.

16. Méthode selon l'une des revendications 1 ou 2, dans laquelle l'épaisseur (y) de l'anneau est de 0,50 mm et l'angle conique (N) est compris entre environ 24° et 30°.

17. Méthode selon l'une des revendications 1 ou 2, dans laquelle l'épaisseur (y) de l'anneau est de 0,55 mm et l'angle conique (N) est compris entre environ 23,5° et 29,5°.
